# EUROPEAN PATENT SPECIFICATION

(11) **EP 2 118 095 B1**
(45) Date of publication and mention of the grant of the patent: **03.11.2010**
(21) Application number: 08715423.3
(22) Date of filing: 25.02.2008
(51) Int. Cl.: C07D 413/06

(54) **A METHOD FOR THE PREPARATION OF ZOLMITRIPTAN**
VERFAHREN ZUR HERSTELLUNG VON ZOLMITRIPTAN
PROCÉDÉ DE PRÉPARATION DE ZOLMITRIPTAN

(30) Priority: 26.02.2007 CZ 20070158
(43) Date of publication of application: 18.11.2009
(73) Proprietor: Zentiva, k.s., 102 37 Praha 10 (CZ)
(72) Inventor: VOSLAR Michal, 103 00 raha 10 (CZ); ZATOPKOVA Monika, 198 00 Praha 9 (CZ); BRUSOVA Hana, 197 00 Praha 9 (CZ); KRUMBHOLCOVA Lucie, 276 01 Melnik (CZ)
(74) Representative: Jirotkova, Ivana
(86) International application number: PCT/CZ2008/000022
(87) International publication number: WO 2008/104135

(56) References cited:
- WO-A-91/18897
- WO-A-97/06162
- WO-A-2005/105792
- WO-A-2006/055964

## Description

### Technical Field

The invention deals with a method of purification of (*S*)-4-[{3-[2-(dimethylaimno)ethyl]-1H-indol-5-yl}methyl]-2-oxazolidinone (zolmitriptan). Zolmitriptan belongs to the group of triptans used for the treatment of migraine.

### Background Art

Synthesis of zolmitriptan consists of three generally known reactions (WO97/06162): diazotation of (S)-4-(4-aminobenzyl)-1,3-oxazolidin-2-one of formula I with a nitrite in the environment of diluted hydrochloric acid, subsequent reduction of the resulting diazonium salt to (*S*)-4-(4-hydrazinobenzyl)-1,3-oxazolidin-2-one of formula IV in an acidic aqueous solution and finally its condensation with 4,4-diethoxy-N,N-dimetylbutylamine of formula II, which, in the acidic environment, according to Fischer, provides the indole skeleton with a mechanism analogous to benzidine rearrangement - in this case zolmitriptan of formula III. The whole synthesis is carried out in an aqueous environment and does not require isolation of intermediate products. 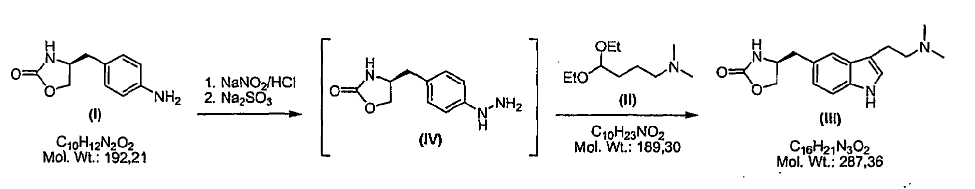 The raw product in the form of the base is usually recovered from the reaction mixture after its alkalization (NaOH/water) by repeated extraction with an organic solvent, generally ethyl acetate (WO 97/06162). After concentration of the extracts to a fraction of the initial volume crystalline raw zolmitriptan is obtained in the form of a solvate with ethyl acetate, which is purified through re-crystallization from an ethanol-ethyl acetate mixture and subsequently desolvated by stirring in aqueous acetone. The product - zolmitriptan base - is finally removed by filtration and washed with ethyl acetate.

With regard to relatively low solubility of zolmitriptan in ethyl acetate the above mentioned extraction method of isolation requires considerable volumes of the extraction agent (ca. 25-fold of the final product) and their subsequent evaporation. To say nothing of the energy and time demands of such a method, during concentrating in a larger scale the product is also subject to considerable heat load which is, with regard to the increased temperature and character of the solvent, even multiplied by the mutual reactivity of the product (basic amine) with the solvent (ester) and also by hydrolysis of the product and solvent caused by the alkaline aqueous environment during the extractions!

WO 91/18897 discloses various salts of zolmitriptan, among them the hydrochloric acid. In WO 2006/055964 the crude product zolmitriptan is suspended in a HCl solution to obtain a pH of 0.5-1, brought directly to pH 7 by addition of potassium carbonate and extracted. In WO 2005/105792 zolmitriptan is purified by dissolution in isopropanol and precipitation by addition of n-heptane.

### Disclosure of Invention

The invention consists in a new method for the preparation of (*S*)-4-[{3-[2-(dimethylamino)ethyl]-1H-indol-5-yl}methyl]-2-oxazolidinone (zolmitriptan), which comprises isolation of a crystalline salt, especially of zolmitriptan hydrochloride.
Another aspect of the invention includes the crystalline hydrochloride of (*S*)4-[{3-[2-(dimethylamino)ethyl]-1H-indol-5-yl}methyl]-2-oxazolidinone (zolmitriptan), which can be used as a useful intermediate of the synthesis, as well as an effective component of a pharmaceutically useful composition.

### Description of Invention

It has been demonstrated experimentally that raw zolmitriptan hydrochloride, prepared by condensation of (*S*)-4-(4-hydrazinobenzyl)-1,3-oxazolidin-2-one of formula IV with 4,4-diethoxy-N,N-dimetylbutyl-amine of formula II in an acidic aqueous environment, can be preferably salted out at a reduced temperature by a considerable increase of the ion strength of the mixture, namely by adding a salt from the group of alkali metal chlorides, preferably NaCl.
Crystalline zolmitriptan hydrochloride is a stable salt, which can be transformed to the free base that is commonly used in the pharmaceutical industry or used directly for the preparation of the medicament, or transformed to another pharmaceutically acceptable salt.

Suitable for the purposes of efficient purification of the product is the crystalline form, characterized by the X-ray diffraction pattern (λCuKα=1.5418 A) in the range of 4 - 40° 2θ with the increment of 0.008 and the typical 2 theta signals: 7.36; 17.91; 18.59; 19.17; 19.40; 23.05.

The crystalline form the reflections of which are summarized in the table below appears to be especially advantageous:

**Table 1.**

| Zolmitriptan.HCl | | |
|---|---|---|
| 2 theta | d | Irel |
| 7.12 | 12.40 | 14.86 |
| 7.36 | 12.00 | 23.61 |
| 12.96 | 6.83 | 12.05 |
| 15.78 | 5.61 | 28.88 |
| 16.01 | 5.53 | 33.55 |
| 17.91 | 4.95 | 83.08 |
| 18.27 | 4.85 | 52.77 |
| 18.59 | 4.77 | 96.48 |
| 18.81 | 4.71 | 39.52 |
| 19.17 | 4.63 | 100.00 |
| 19.40 | 4.57 | 58.44 |
| 21.58 | 4.12 | 27.54 |
| 21.99 | 4.04 | 51.71 |
| 22.43 | 3.96 | 33.89 |
| 23.05 | 3.86 | 64.52 |
| 23.33 | 3.81 | 20.69 |
| 25.03 | 3.56 | 8.74 |
| 25.18 | 3.53 | 12.89 |
| 25.37 | 3.51 | 11.50 |
| 25.89 | 3.44 | 9.10 |
| 26.09 | 3.41 | 17.94 |
| 26.46 | 3.37 | 10.71 |
| 26.63 | 3.34 | 10.51 |
| 28.16 | 3.17 | 7.67 |
| 28.71 | 3.11 | 10.13 |
| 29.32 | 3.04 | 8.67 |
| 30.16 | 2.96 | 11.01 |
| 30.24 | 2.95 | 9.16 |
| 31.69 | 2.82 | 9.29 |
| 36.65 | 2.45 | 11.37 |

The crystalline form of zolmitriptan hydrochloride prepared in the above-described preferable method is further characterized by a melting temperature in the interval of 148 to 154 °C and also by an endotherm with the peak at 142 ± 3 °C and the onset at 127 ± 3 °C, measured in an N₂ stream, in a DSC record at the heating rate of 10°C/min.
Zolmitriptan in the free base form, which is insoluble in water, is released from the salt by gradual addition of an alkali metal carbonate or hydroxide, e.g. K₂CO₃ or NaOH, etc., to an aqueous solution of the hydrochloride or its solution in an alcohol-water mixture. The base is removed by filtration and washed with aqueous alcohol and water, or it is stirred up in purified water. After drying at temperatures of about 45°C the obtained zolmitriptan in the base form can be optionally re-purified by re-crystallization from an ethanol-water mixture. The resulting white or almost white product (HPLC ∼100.0 %) corresponds, according to the X-ray analysis, to form A, the same one as can be prepared with the method in accordance with patent application no. WO 97/06162.
Advantages of the above mentioned newly developed isolation and purification process include the speed, low price of input materials, a high yield and quality of the obtained product and, above all, minimum impacts on the environment since except a small volume of ethanol no organic solvents are used in the whole process. At the same time the process has managed to remove the above mentioned problems that accompany the common isolation method based on extractions of the product with ethyl acetate.

### Brief Description of Drawings

Figure 1 shows an X-ray diffraction pattern of the HCl salt of zolmitriptan.
Figure 2 shows a DSC record of the HCl salt of zolmitriptan.

### Examples

### Example 1 - Fischer indole reaction

The diluted reaction mixture containing (*S*)-4-(4-hydrazinobenzyl)-1,3-oxazolidin-2-one prepared by diazotation 1.75 kg of the amine (I) and reduction in accordance with the above mentioned scheme is heated up to ca. 90°C. 1.75 kg of 4,4-diethoxy-N,N-dimethylbutylamine of formula II is weighed. The weighed acetal (II) is added to the reaction mixture, which is then brought to moderate reflux at ca. 98°C and left to react being stirred under a reflux condenser for 2.5 hours. After 2.5 hours from the start of the reflux the heating of the mixture is switched off and the reaction mixture is cooled to the laboratory temperature.

### Example 2 - Isolation of raw ZOLMITRIPTAN hydrochloride

The reaction mixture is cooled down to ca. 10°C and about 9 kg of NaCl is added during stirring. The mixture is stirred at this temperature for another ca. 45 min. The salted out zolmitriptan hydrochloride is then removed by filtration and washed with ca. 1.7 litres of saturated NaCl solution.

### Example 3 - Transforming the ZOLMITRIPTAN hydrochloride to the base

The zolmitriptan hydrochloride salted out from the reaction mixture in accordance with Example 4 is dissolved in ca. 12 litres of water under stirring and about 2.5 litres of ethanol are added. 1.0 kg of potassium carbonate is added in several doses to the solution under stirring at the temperature of ca. 25°C. 30 mins after the last addition the suspension of the product is cooled to 10 - 15°C while being stirred and the product is removed by filtration after approximately 1 hour of stirring. The filtration cake is repeatedly washed with ca. 2 litres of purified water (or the filtered base is re-suspended and stirred up in purified water) and left to dry under normal pressure at the temperature of about 45°C to the constant weight.
The product may be optionally re-purified by re-crystallization from an ethanol-water mixture.

### Example 4 - Purification of ZOLMITRIPTAN hydrochloride

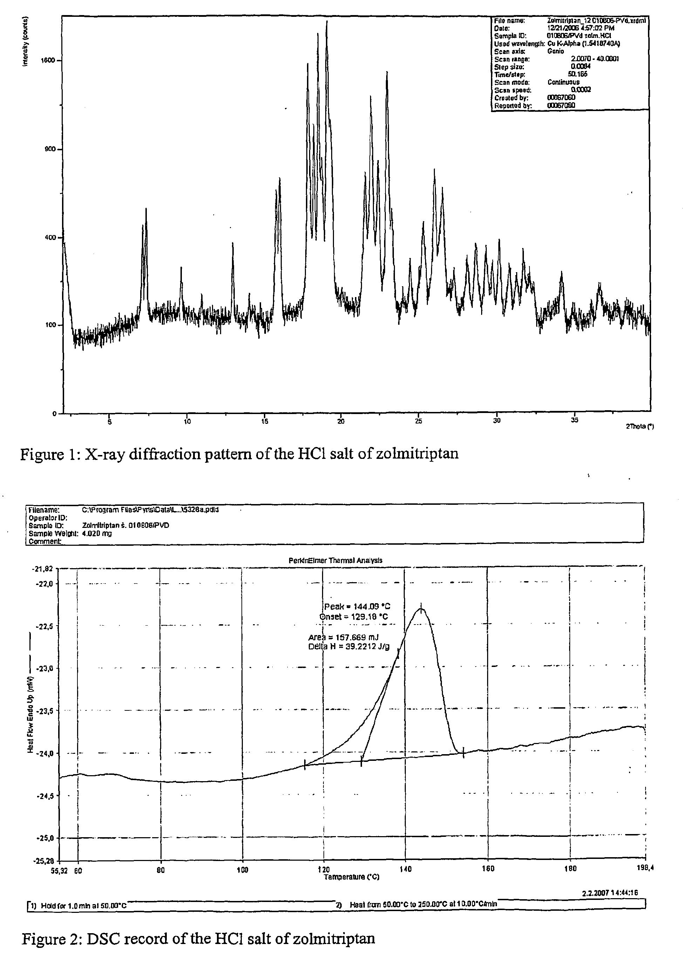

Raw zolmitriptan hydrochloride is re-purified by conversion to the base in aqueous alcohol in accordance with Example 3. Under stirring the base is then dissolved in ca. 8 litres of 2% HCl and solid zolmitriptan hydrochloride is salted out again from this aqueous solution by the addition of about 2 kg of NaCl at the temperature of ca. 10°C. Approximately after 30 minutes of stirring the product is removed by filtration and washed with ice-cold water, or stirred up in ice-cold water. Pure zolmitriptan hydrochloride is dried in vacuo at the laboratory temperature for about 8 hours and then to the constant weight at ca. 50°C.
The obtained zolmitriptan hydrochloride salt was characterized by the X-ray powder diffraction method (Fig. 1) and the DSC method (Fig. 2).

## Claims

1. A method for the preparation of (*S*)-4-[{3-[2-(dimethylamino)ethyl]-1H-indol-5-yl}methyl]-2-oxazolidinone (zolmitriptan) of formula III 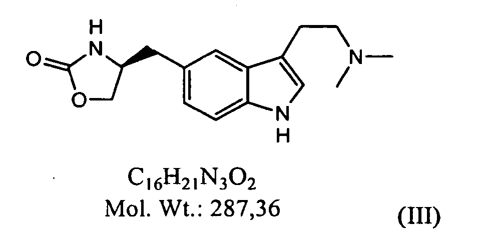 or its pharmaceutically acceptable salts, **characterized in that** it comprises
a) Preparation of zolmitriptan hydrochloride by reacting of (*S*)-4-(4-hydrazinobenzyl)-1,3-oxazolidin-2-one hydrochloride with 4,4-diethoxy-N,N-dimethylbutylamine of formula II by the Fischer indole reaction;
b) Salting out of zolmitriptan hydrochloride from the reaction mixture after the Fischer indole synthesis, at a reduced temperature by addition of a salt from the group of alkali metal chlorides, preferably NaCl;
c) Re-purification of the raw zolmitriptan hydrochloride by conversion to the base by addition of an alkali metal carbonate or hydroxide, e.g. K₂CO₃ or NaOH, etc., in aqueous alcohol, and recovering an aqueous solution of the hydrochloride by dissolution of the filtered base in a diluted acid, e.g. HCl;
d) Isolation of pure zolmitriptan hydrochloride in the crystalline form by salting out from the acidic aqueous solution;
e) Desalting of zolmitriptan hydrochloride by stirring up in ice-cold water; and
f) Converting the hydrochloride to the desired substance of formula III of high purity by the effect on an alkali metal carbonate or hydroxide.

2. The method in accordance with claim 1, **characterized in that** it further comprises conversion of zolmitriptan hydrochloride to another pharmaceutically acceptable salt.

3. The crystalline zolmitriptan hydrochloride obtainable by the method of claim 1, having a melting temperature in the interval of 148 - 154 °C.

4. The crystalline zolmitriptan hydrochloride in accordance with claim 3, **characterized by** a DSC record displaying one endotherm with the peak at 142 ± 3 °C and onset at 127 ± 3 °C in the measured interval of 50 - 200 °C at the heating rate of 10 °C/min, measured in an N₂ stream.

5. The crystalline zolmitriptan hydrochloride in accordance with claim 3, **characterized by** the X-ray diffractogram (λCuKα=1.5418 A) in the range of 4 - 40° with the increment of 0.008 with the typical 2 theta signals: 7.36; 17.91; 18.59; 19.17; 19.40; 23.05.

## Patentansprüche

1. Verfahren zur Herstellung von (S)-4-[{3-[2-(Dimethylamino)ethyl]-1H-indol-5-yl}methyl]-2-oxazolidinon (Zolmitriptan) der Formel (III): 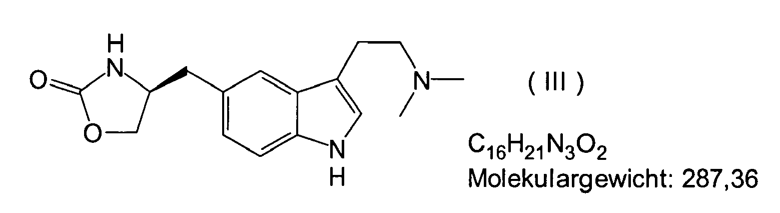 oder seines pharmazeutisch annehmbaren Salzes, **dadurch gekennzeichnet, dass** es umfasst:
(a) Herstellung von Zolmitriptan-Hydrochlorid durch Umsetzung von (S)-4-(4-Hydrazinobenzyl)-1,3-oxazolidin-2-on-Hydrochlorid mit 4,4-Diethoxy-N,N-dimethylbutylamin der Formel (II) mittels Fischer-Indolreaktion;
(b) Aussalzen des Zolmitriptan-Hydrochlorids aus der Reaktionsmischung nach der Fischer-Indolsynthese bei reduzierter Temperatur durch Zugabe eines Salzes der Gruppe von Alkalimetallchloriden, vorzugsweise NaCl;
(c) erneute Reinigung des rohen Zolmitriptan-Hydrochlorids durch Umwandlung zur Base durch Zugabe eines Alkalimetallcarbonats oder -hydroxids, z.B. K₂CO₃ oder NaOH, etc., in wässrigem Alkohol und Rückgewinnung der wässrigen Hydrochloridlösung durch Auflösen der filtrierten Base in verdünnter Säure, z.B. HCl;
(d) Isolieren des reinen Zolmitriptan-Hydrochlorids in kristalliner Form durch Aussalzen aus der sauren wässrigen Lösung;
(e) Entsalzen des Zolmitriptan-Hydrochlorids durch Rühren in eiskaltem Wasser; und
(f) Umwandeln des Hydrochlorids zu der gewünschten Substanz der Formel (III) hoher Reinheit durch die Wirkung von Alkalimetallcarbonat oder -hydroxid.

2. Verfahren gemäss Anspruch 1, **dadurch gekennzeichnet, dass** es ferner die Umwandlung von Zolmitriptan-Hydrochlorid in ein anderes pharmazeutisch annehmbares Salz umfasst.

3. Kristallines Zolmitriptan-Hydrochlorid, erhältlich nach dem Verfahren gemäss Anspruch 1 mit einem Schmelzpunkt in Intervallen von 148 bis 154°C.

4. Kristallines Zolmitriptan-Hydrochlorid gemäss Anspruch 3, **dadurch gekennzeichnet, dass** eine DSC-Aufzeichnung eine Endotherme mit einem Peak bei 143 ± 3°C und einem Anfang bei 127 ± 3°C in dem gemessenen Intervall von 50 bis 200°C bei einer Erwärmungsgeschwindigkeit von 10°C/min, gemessen in einem N₂-Strom, zeigt.

5. Kristallines Zolmitriptan-Hydrochlorid gemäss Anspruch 3, **gekennzeichnet durch** ein Röntgenstrahlen-Diffraktogramm (λCuKα = 1,5418 Å) im Bereich von 4 bis 40° mit einer Zunahme von 0,008 mit den typischen 2θ-Signalen: 7,36; 17,91; 18,59; 19,17; 19,40; 23,05.

## Revendications

1. Un procédé pour la préparation de (*S*)-4-[{3-[2-(diméthylamino)-éthyl]-1H-indol-5-yl}-méthyl]-2-oxazolidinone (zolmitriptan) de formule III 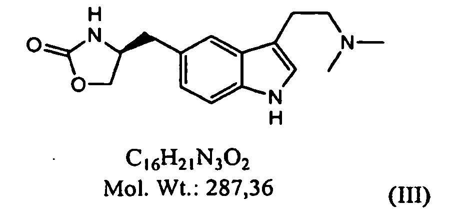 ou des sels pharmaceutiquement acceptables en dérivant, **caractérisé en ce qu'**il comprend :
a) la préparation du chlorhydrate de zolmitriptan par réaction de chlorhydrate de (*S*)-4-(4-hydrazinobenzyl)-1,3-oxazolidin-2-one avec la 4,4-diéthoxy-N,N-diméthylbutyl-amine de formule II par la réaction d'indole de Fischer;
b) Le relargage du chlorhydrate de zolmitriptan contenu dans le mélange réactionnel après la synthèse d'indole de Fischer, à une température réduite, par addition d'un sel du groupe des chlorures de métal alcalin, de préférence NaCl;
c) La re-purification du chlorhydrate de zolmitriptan brut par conversion à l'état de base par addition d'un carbonate ou hydroxyde de métal alcalin, par exemple K₂CO₃ ou NaOH, etc., dans une solution hydroalcoolique et la récupération d'une solution aqueuse de chlorhydrate par dissolution de la base filtrée dans un acide dilué par exemple HCl;
d) L'isolation du chlorhydrate de zolmitriptan pur sous forme cristallisée par relargage de la solution aqueuse acide,
e) Le désalage du chlorhydrate de zolmitriptan par agitation dans de l'eau glacée; et
f) La conversion du chlorhydrate en le produit de formule III présentant une pureté élevée par action du carbonate ou de l'hydroxyde de métal alcalin.

2. Le procédé selon la revendication 1, **caractérisé en ce que** il comprend en outre la conversion du chlorhydrate de zolmitriptan en un autre sel pharmaceutiquement acceptable.

3. Le chlorhydrate de zolmitriptan cristallin obtenu par le procédé selon la revendication 1, présentant une température de fusion comprise entre 148 et 154°C.

4. Le chlorhydrate de zolmitriptan cristallin selon la revendication 3, **caractérisé par** un enregistrement DSC présentant un endotherme ayant un pic à 142 ± 3°C et un début à 127 ± 3°C dans l'intervalle mesuré de 50 à 200°C à une vitesse de chauffage de 10°C/min, mesuré dans un courant de Nu₂

5. Le chlorhydrate de zolmitriptan cristallin selon la revendication 3, **caractérisé par** un diffractogramme aux rayons X (λCuKα= 1.5418 Å) de l'ordre de 4 à 40° avec un incrément de 0,008 avec les signaux 2 théta typiques à: 7,36; 17,91; 18,59; 19,17; 19,40; 23,05.
